# EUROPEAN PATENT SPECIFICATION

(11) **EP 1 373 216 B1**
(45) Date of publication and mention of the grant of the patent: **15.12.2004**
(21) Application number: 02726190.8
(22) Date of filing: 18.03.2002
(51) Int. Cl.: C07D 231/06, C07D 401/04, A61K 31/415, A61P 25/00

(54) **4,5-DIHYDRO-1H-PYRAZOLE DERIVATIVES HAVING CB1-ANTAGONISTIC ACTIVITY**
4,5-DIHYDRO-1H-PYRAZOLDERIVATE MIT CB1-ANTAGONISTISCHER WIRKUNG
DERIVES DE 4,5-DIHYDRO-1H-PYRAZOLE AYANT UNE ACTIVITE D'ANTAGONISTE DE CB 1

(30) Priority: 22.03.2001 EP 01201062
(43) Date of publication of application: 02.01.2004
(73) Proprietor: Solvay Pharmaceuticals B.V., 1381 CP Weesp (NL)
(72) Inventor: LANGE, Josephus, H.M., NL-1381 CP Weesp (NL); KRUSE, Cornelis, G, NL-1381 CP Weesp (NL); TIPKER, Jacobus, NL-1381 CP Weesp (NL); HOOGENDOORN, Jan, NL-1381 CP Weesp (NL)
(74) Representative: Verhage, Marinus, Dr.
(86) International application number: PCT/EP2002/003079
(87) International publication number: WO 2002/076949

(56) References cited:
- EP-A- 0 173 323
- US-A- 4 070 365
- US-A- 5 624 941
- PERTWEE, R. G.: "Pharmacology of Cannabinoid Receptor Ligands" CURRENT MED. CHEM., vol. 6, 1999, pages 635-664, XP000923352 cited in the application
- LANGE J.H.M. ET AL: 'Synthesis, Biological Properties, and Molecular Modelling Investigations of Novel 3,4-Diarylpyrazolines as Potent and Selective CB1 Cannabinoid Receptor Antagonists' J.MED.CHEM. vol. 47, no. 3, 2004, pages 627 - 643

## Description

The present invention relates to a group of novel enantiomers of 4,5-dihydro-1H-pyrazole derivatives having S configuration at the 4-position of their 4,5-dihydropyrazole ring, to methods for the preparation of these compounds, and to pharmaceutical compositions containing one or more of these compounds as an active component.
The above mentioned (4S)-4,5-dihydro-1H-pyrazoles are potent Cannabis-1 (CB₁) receptor antagonists with utility for the treatment of psychiatric and neurological disorders.

Cannabinoids are present in the Indian hemp *Cannabis Sativa L*. and have been used as medicinal agents for centuries (Mechoulam, R.; Feigenbaum, J.J. *Prog. Med. Chem.* **1987**, *24*, 159). However, only within the past ten years the research in the cannabinoid area has revealed pivotal information on cannabinoid receptors and their (endogenous) agonists and antagonists. The discovery and the subsequent cloning of two different subtypes of Cannabinoid receptors (CB₁ and CB₂) stimulated the search for novel cannabinoid receptor antagonists (Munro, S.; Thomas, K.L.; Abu-Shaar, M. *Nature* **1993**, *365*, 61. Matsuda, L.A.; Bonner, T.I. *Cannabinoid Receptors,* Pertwee, R.G. Ed. **1995**, 117, Academic Press, London). In addition, pharmaceutical companies became interested in the development of cannabinoid drugs for the treatment of diseases connected with disorders of the cannabinoid system. The wide distribution of CB₁ receptors in the brain, in combination with the strictly peripheral localisation of the CB₂ receptor, makes the CB₁ receptor a very interesting molecular target for CNS-directed drug discovery in the areas of both psychiatric and neurological disorders (Consroe, P. *Neurobiology of Disease* **1998**, *5*, 534. Pop, E. *Curr*. *Opin. In CPNS Investigational Drugs* **1999**, *1*, 587. Greenberg, D.A. *Drug News Perspect.* **1999**, *12*, 458). Hitherto, three types of distinct CB₁ receptor antagonists are known. Sanofi disclosed their diarylpyrazole congeners as selective CB₁ receptor antagonists. A representative example is SR-141716A, which is currently undergoing Phase II clinical development for psychotic disorders (Dutta, A.K.; Sard, H.; Ryan, W.; Razdan, R.K.; Compton, D.R.; Martin, B.R. *Med. Chem. Res.* **1994**, *5*, 54. Lan, R.; Liu, Q.; Fan, P.; Lin, S.; Fernando, S.R.; McCallion, D.; Pertwee, R.; Makriyannis, A. *J. Med. Chem*. **1999**, *42*, 769. Nakamura-Palacios, E.M.; Moerschbaecher, J.M.; Barker, L.A. *CNS Drug Rev.* **1999**, *5*, 43). Aminoalkylindoles have been disclosed as CB₁ receptor antagonists. A representative example is lodopravadoline (AM-630), which was introduced in 1995. AM-630 is a CB₁ receptor antagonist, but sometimes behaves as a weak partial agonist (Hosohata, K.; Quock, R.M.; Hosohata, Y.; Burkey, T.H.; Makriyannis, A.; Consroe, P.; Roeske, W.R.; Yamamura, H.I. *Life* Sc. **1997,** *61*, PL115). More recently, researchers from Eli Lilly described aryl-aroyl substituted benzofurans as selective CB₁ receptor antagonists (*e.g.* LY-320135) (Felder, C.C.; Joyce, K.E.; Briley, E.J.; Glass, M.; Mackie, K.P.; Fahey, K.J.; Cullinan, G.J.; Hunden, D.C.; Johnson, D.W.; Chaney, M.O.; Koppel, G.A.; Brownstein, M. *J. Pharmacol*. *Exp. Ther.* **1998,** *284*, 291). Recently, 3-alkyl-5,5'-diphenylimidazolidinediones were described as cannabinoid receptor ligands, which were indicated to be cannabinoid antagonists (Kanyonyo, M.; Govaerts, S.J.; Hermans, E.; Poupaert, J.H., Lambert, D.M. *Biorg. Med*.*Chem*. *Lett.* **1999**, *9*, 2233). Interestingly, many CB₁ receptor antagonists have been reported to behave as inverse agonists *in vitro* (Landsman, R.S.; Burkey, T.H.; Consroe, P.; Roeske, W.R.; Yamamura, H.I. *Eur. J. Pharmacol*. **1997,** *334*, R1). Recent reviews provide a nice overview of the current status in the cannabinoid research area (Mechoulam, R.; Hanus, L.; Fride, E. *Prog. Med*. *Chem*. **1998,** *35*, 199. Lambert, D.M. *Curr*. *Med*. *Chem.* **1999,** *6*, 635. Mechoulam, R.; Fride, E.; Di Marzo, V. *Eur*. *J. Pharmacol*. **1998,** *359*, 1).

It has now surprisingly been found that the novel enantiomers of 4,5-dihydro-1H-pyrazole derivatives having S configuration at the 4-position of their 4,5-dihydro pyrazole ring of the formula (I), tautomers thereof and salts thereof 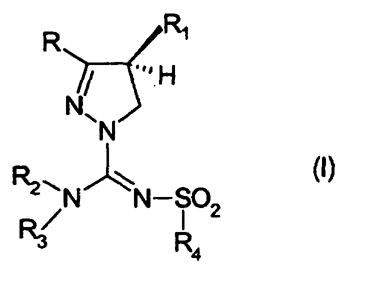 wherein
- R and R₁ are the same or different and represent 3-pyridyl or 4-pyridyl, or phenyl which may be substituted with halogen or methoxy,
- R₂ and R₃ are the same or different and represent hydrogen, alkyl (1-3 C) or dimethylamino
- R₄ represents phenyl which may be substituted with 1, 2 or 3 substituents selected from the group halogen, trifluoromethyl, methoxy and alkyl (1-3 C) are much more potent and selective antagonists of the cannabis CB₁-receptor, than the correspondence R-enantiomer.

Due to the potent CB₁ antagonistic activity the compounds according to the invention are suitable for use in the treatment of psychiatric disorders such as psychosis, anxiety, depression, attention deficits, memory disorders and appetite disorders, obesity, neurological disorders such as dementia, distonia, Parkinson's disease, Alzheimer's disease, epilepsy, Huntington's disease, Tourette's syndrome, cerebral ischaemia, as well as for the treatment of pain disorders and other CNS-diseases involving cannabinoid neurotransmission, and in the treatment of gastrointestinal disorders and cardiovascular disorders.

The affinity of the compounds of the invention for cannabinoid CB₁ receptors was determined using membrane preparations of Chinese hamster ovary (CHO) cells in which the human cannabis CB₁ receptor is stably transfected in conjunction with [3H]CP-55,940 as radioligand. After incubation of a freshly prepared cell membrane preparation with the [3H]-ligand, with or without addition of compounds of the invention, separation of bound and free ligand was performed by filtration over glassfiber filters. Radioactivity on the filter was measured by liquid scintillation counting.

The cannabinoid CB₁ antagonistic activity of compounds of the invention was determined by functional studies using CHO cells in which human cannabinoid CB₁ receptors are stably expressed. Adenylyl cyclase was stimulated using forskolin and measured by quantifying the amount of accumulated cyclic AMP. Concomitant activation of CB₁ receptors by CB₁ receptor agonists (*e.g.* CP-55,940 or (R)-WIN-55,212-2) can attenuate the forskolin-induced accumulation of CAMP in a concentration-dependent manner. This CB₁ receptor-mediated response can be antagonised by CB₁ receptor antagonists such as the compounds of the invention.

The invention relates both to the E isomer, Z isomer and E/Z mixtures of compounds having formula (I).

The compounds can be brought into forms suitable for administration by means of usual processes using auxiliary substances and/or liquid or solid carrier materials.

The compounds of the invention having formula (III) (*vide infra*) can be obtained according to methods known, for example: a) EP 0021506; b) DE 2529689.

A suitable synthesis for the racemic compounds according to the present invention is the following:

### Synthesis route A

### Step 1 of route A

Reaction of a compound having formula (III) 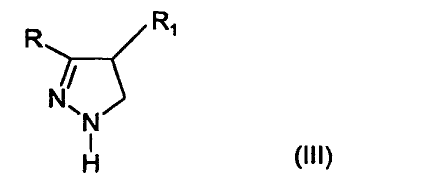 with a compound having formula (IV) 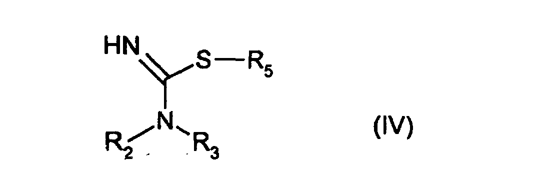 wherein R₅ represents a lower alkyl group, such as for example 2-methyl-2-thiopseudourea, or with a suitable salt form thereof in the presence of a base. This reaction gives a 4,5-dihydro-1H-pyrazole-1-carboxamidine derivative having formula (V) 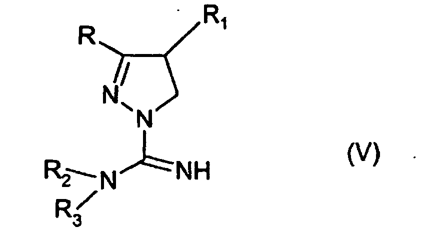 wherein the symbols have the meanings as mentioned above. Compounds having formula (V) wherein R, R₁, R₂ and R₃ have the meaning as described herein above for compound (I) are new.

Alternatively, a compound having formula (III) is reacted with a so-called guanylating agent. Examples of such guanylating agents are 1H-pyrazole-1-carboxamidine and its salts (for example the hydrochloride salt) and 3,5-dimethyl-1H-pyrazole-1-carboxamidine and its salts (for example the nitrate salt) and the like. This reaction gives a carboxamidine derivative having formula (V).

Alternatively, a compound having formula (III) is reacted with a so-called protected guanylating agent. Examples of such protected guanylating agents are N-(benzyloxycarbonyl)-1H-pyrazole-1-carboxamidine, N-(*tert*-butoxycarbonyl)-1H-pyrazole-1-carboxamidine and N,N'-bis-(*tert*-butoxycarbonyl)-1H-pyrazole-1-carboxamidine and the like. This reaction gives after deprotection a compound having formula (V).

### Step 2 of route A

The compound having formula (V) is reacted with an optionally substituted compound of the formula R₄-SO₂X, wherein R₄ has the above mentioned meaning and X represents a halogen atom. This reaction is preferably carried out in the presence of a base, such as triethylamine in an aprotic solvent, such as acetonitrile.

### Synthesis route A1

### Step 1 of route A1

Reaction of a compound having formula (III) 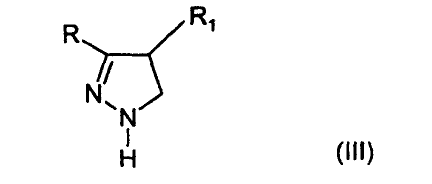 with a thioisocyanate derivative having formula (VI).

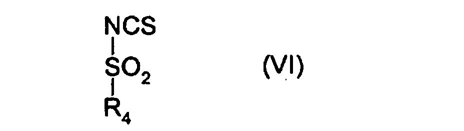

This reaction is preferably carried out in an inert organic solvent, such as for example acetonitrile.

This reaction gives a thiocarboxamide derivative having formula (VII). Compounds having formula (VII) wherein R, R₁ and R₄ have the meaning as described herein above for compound (I) are new.

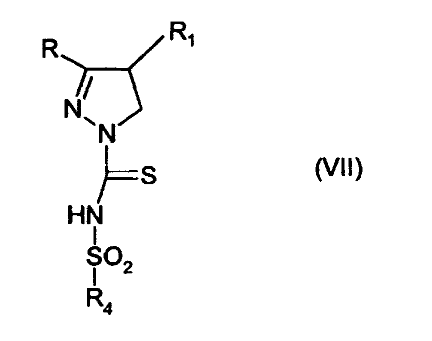

### Step 2 of route A1

Reaction of a compound having formula (VII) with an amine in the presence of a mercury(II) salt, such as for example HgCl₂, gives a compound having formula (I) This reaction is preferably carried out in a polar organic solvent, such as for example acetonitrile.

### Synthesis route A2

### Step 1 of route A2

Reaction of a compound having formula III 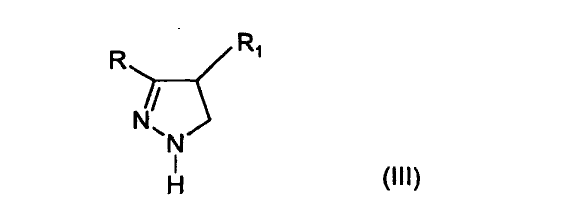 with a carbamate ester derivative having formula (VIII). 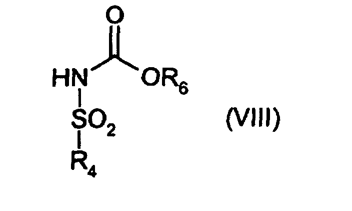 wherein R₆ represents a lower alkyl group, for example methyl.

This reaction is preferably carried out in an inert organic solvent, such as for example 1,4-dioxane.
This reaction gives a 4,5-dihydropyrazole-1-carboxamide derivative having formula (IX). Compounds having formula (IX) wherein R, R₁ and R₄ have the meaning as described herein above for compound (I) are new.

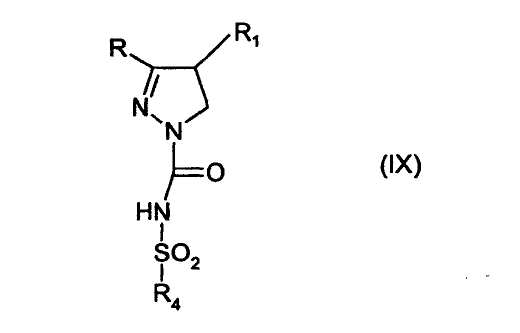

### Step 2 of route A2

Reaction of a compound having formula (IX) with a halogenating agent, such as for example PCl₅, gives a 4,5-dihydropyrazole-1-carboxirnidoyl halogenide derivative having formula (X). 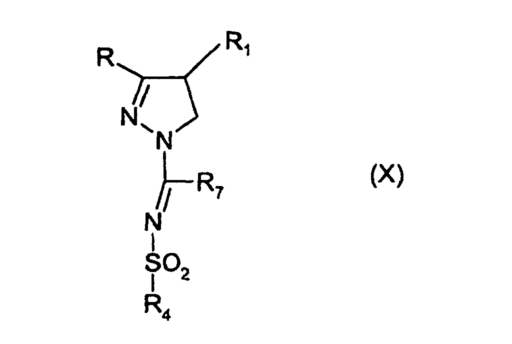 wherein R₇ represents a halogen atom, such as for example chloro. This reaction is preferably carried out in an inert organic solvent, such as for example chlorobenzene.
Compounds having formula (X) wherein R, R₁ and R₄ have the meaning as described herein above for compound (I) and wherein R₇ represents a halogen atom are new.

### Step 3 of route A2

Reaction of a compound having formula (X) with an amine gives a compound having formula (I).
This reaction is preferably carried out in an inert organic solvent, such as for example dichloromethane.

### Synthesis route A3

### Step 1 of route A3

Reaction of a compound having formula III 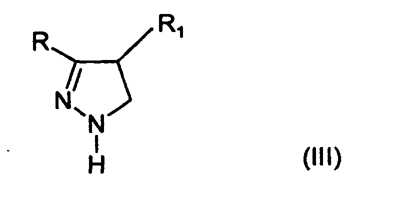 with a dithioimidocarbonic ester derivative having formula (XI). 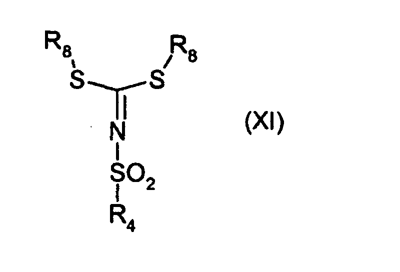 wherein R₈ represents a C₁₋₃ alkyl group.
This reaction is preferably carried out in a polar organic solvent, such as for example acetonitrile.

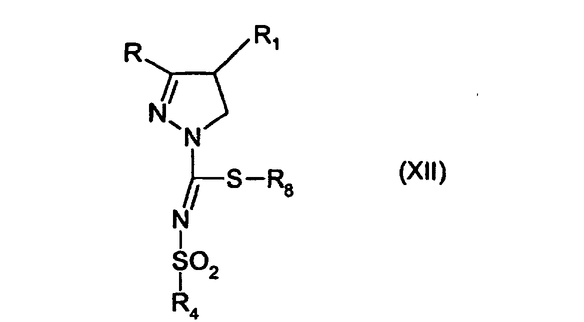

This reaction gives a carboximidothioic ester derivative having fomula (XII).

Compounds having formula (XII) wherein R, R₁ and R₄ have the meaning as described herein above for compound (I) and wherein R₈ represents a C₁₋₃ alkyl group are new.

### Step 2 of route A3

Reaction of a compound having formula (XII) with an amine gives a compound having formula (I).
This reaction is preferably carried out in a polar organic solvent, such as for example methanol.

### Example I

### 3-(4-Chlorophenyl)-4,5-dihydro-N-((4-fluorophenyl)sulfonyl)-4-phenyl-1H-pyrazole-1-carboxamidine

**Part A:** A stirred mixture of 3-(4-chlorophenyl)-4,5-dihydro-4-phenyl-1H-pyrazole (5.13 gram, 20.0 mmol), 2-methyl-2-thiopseudourea hydroiodide (5.00 gram, 23.0 mmol) and pyridine (10 ml) is heated at 110 °C for 1 hour. After one night standing at room temperature diethyl ether is added and the precipitate is collected by filtration. This precipitate is washed three times with diethyl ether portions to afford a solid (9 gram). Melting point: -230 °C. This solid is dissolved in methanol (20 ml). To the resulting solution is successively added a 2N sodium hydroxide solution (12 ml) and water (200 ml). The formed precipitate is collected by filtration, washed two times with diethyl ether and successively with diisopropyl ether. The resulting solid is dried *in vacuo* to yield 3-(4-chlorophenyl)-4,5-dihydro-4-phenyl-1H-pyrazole-1-carboxamidine (5.1 gram, 88 % yield). Melting point: 187-189 °C.

**Part B:** To a stirred mixture of 3-(4-chlorophenyl)-4,5-dihydro-4-phenyl-1H-pyrazole-1-carboxamidine (0.50 gram, 1.68 mmol) and 4-fluorophenylsulfonyl chloride (0.34 gram, 1.75 mmol) in acetonitrile (10 ml) is added N,N-dimethyl-4-aminopyridine (0.020 gram, 0.175 mmol) and triethylamine (1 ml). The resulting solution is stirred at room temperature for 30 minutes. After addition of a 2N sodium hydroxide solution and extraction with ethylacetate (400 ml), the ethylacetate layer is concentrated *in vacuo.* The resulting crude residue is further purified by means of flash chromatography (petroleum ether/diethyl ether = 1/1 (v/v), followed by ethylacetate). Subsequent concentration *in vacuo* affords solid 3-(4-chlorophenyl)-4,5-dihydro-N-((4-fluorophenyl)sulfonyl)-4-phenyl-1H-pyrazole-1-carboxamidine (0.55 gram, 72 % yield). Melting point: 214-215 °C

In an analogous manner the compounds having formula (I) listed below have been prepared:
4,5-Dihydro-N-((4-fluorophenyl)sulfonyl)-3-(4-methoxyphenyl)-4-(4-methoxyphenyl)-1H-pyrazole-1-carboxamidine: Melting point: 155-156 °C
4,5-Dihydro-3-(4-methoxyphenyl)-4-(4-methoxyphenyl)-N-((4-methoxyphenyl)sulfonyl)-1H-pyrazole-1-carboxamidine: Melting point: 148-150 °C
3-(4-Chlorophenyl)-4,5-dihydro-4-phenyl-N-((2,4,6-trimethylphenyl)sulfonyl)-1H-pyrazole-1-carboxamidine: Melting point: 221-222 °C

### Example II

### N¹,N¹-Dimethyl-N²-((4-chlorophenyl)sulfonyl)-3-(4-chlorophenyl)-4,5-dihydro-4-phenyl-1H-pyrazole-1-carboxamidine

**Part A**: A stirred mixture of 3-(4-chlorophenyl)-4,5-dihydro-4-phenyl-1H-pyrazole (12.0 gram, 46.8 mmol), [(4-chlorophenyl)sulfonyl]dithioimidocarbonic acid dimethyl ester (CAS: 13068-12-7) (9.20 gram, 31.1 mmol) and triethylamine (15 ml) in acetonitrile (200 ml) is heated at reflux temperature for 20 hours. An additional portion of 3-(4-chlorophenyl)-4,5-dihydro-4-phenyl-1H-pyrazole (12.0 gram, 46.8 mmol) is added and the resulting mixture is heated at reflux temperature for another 16 hours. After concentration *in vacuo*, dichloromethane is added and the resulting solution is washed twice with water and dried over anhydrous Na₂SO₄. After filtration and evaporation *in vacuo* the residue is further purified by flash chromatography (diethyl ether/ petroleum ether = 1/1 (v/v)) to give 3-(4-chlorophenyl)-N-((4-chlorophenyl)sulfonyl)-4,5-dihydro-4-phenyl-1H-pyrazole-1-carboximidothioic acid methyl ester (12.5 gram, 80% yield based on [(4-chlorophenyl)sulfonyl]dithioimidocarbonic acid dimethyl ester) as an amorphous solid.

**Part B:** To a stirred mixture of 3-(4-chlorophenyl)-N-((4-chlorophenyl)sulfonyl)-4,5-dihydro-4-phenyl-1H-pyrazole-1-carboximidothioic acid methyl ester (4.20 gram, 8.30 mmol) in methanol (75 ml) is added dimethylamine (10 ml) and dichloromethane (75 ml) and the resulting solution is stirred at room temperature for 6 hours. Evaporation *in vacuo* and subsequent flash chromatographic purification (diethyl ether/ petroleum ether = 1/1 (v/v), followed by diethyl ether) gives a solid which is further purified by recrystallisation from diisopropyl ether to yield N¹-dimethyl-N²-((4-chloro-phenyl)sulfonyl)-3-(4-chlorophenyl)-4,5-dihydro-4-phenyl-1H-pyrazole-1-carboxamidine (2.63 gram, 63 % yield). Melting point: 182 °C.

In an analogous manner the compounds having formula (I) listed below have been prepared:
N-Methyl-N'-((4-chlorophenyl)sulfonyl)-3-(4-chlorophenyl)-4,5-dihydro-4-(3-pyridyl)-1H-pyrazole-1-carboxamidine. Melting point: 101-105 °C.
N-Methyl-N'-((4-chlorophenyl)sulfonyl)-3-(4-chlorophenyl)-4,5-dihydro-4-(4-pyridyl)-1H-pyrazole-1-carboxamidine. Melting point: 112-115 °C.

### Example III

### N-Methyl-N'-((4-chlorophenyl)sulfonyl)-3-(4-chlorophenyl)-4,5-dihydro-4-phenyl-1H-pyrazole-1-carboxamidine

**Part A:** To a solution of N-((4-chlorophenyl)sulfonyl)carbamic acid methyl ester (CAS: 34543-04-9) (2.99 gram, 12.0 mmol) and pyridine (4 ml) in 1,4-dioxane (20 ml) is added 3-(4-chlorophenyl)-4,5-dihydro-4-phenyl-1H-pyrazole (3.39 gram, 13.2 mmol) and the resulting mixture is stirred for 4 hours at 100 °C. After concentration *in vacuo* the residue is dissolved in dichloromethane, successively washed with water, 1N HCl and water, dried over anhydrous Na₂SO₄, filtered and concentrated *in vacuo* to a volume of 20 ml. Methyl-tert-butyl ether (60 ml) is added and the resulting solution is concentrated to a volume of 20 ml. The formed crystals are collected by filtration and recrystallised from methyl-*tert*-butyl ether to give 3-(4-chlorophenyl)-N-((4-chlorophenyl)sulfonyl)-4,5-dihydro-4-phenyl-1H-pyrazole-1-carboxamide (4.75 gram, 76 % yield) Melting point: 211-214 °C.

**Part B**: A mixture of 3-(4-chlorophenyl)-N-((4-chlorophenyl)sulfonyl)-4,5-dihydro-4-phenyl-1H-pyrazole-1-carboxamide (3.67 gram, 7.75 mmol) and phosphorus pentachloride (1.69 gram, 8.14 mmol) in chlorobenzene (40 ml) is heated at reflux for 1 hour. After thorough concentration *in vacuo,* the formed N-((4-chlorophenyl)sulfonyl)-3-(4-chlorophenyl)-4,5-dihydro-4-phenyl-1H-pyrazole-1-carboximidoyl chloride is suspended in dichloromethane and reacted with cold methylamine (1.5 ml). After stirring at room temperature for 1 hour, the mixture is concentrated *in vacuo.* The residue is crystallised from diethyl ether to give N-methyl-N'-((4-chlorophenyl)sulfonyl)-3-(4-chlorophenyl)-4,5-dihydro-4-phenyl-1H-pyrazole-1-carboxamidine (2.29 gram, 61 % yield). Melting point: 96-98 °C (dec.).

In an analogous manner the compounds having formula (I) listed below have been prepared:
N-Methyl-N'-((3-chlorophenyl)sulfonyl)-3-(4-chlorophenyl)-4,5-dihydro-4-phenyl-1H-pyrazole-1-carboxamidine. Melting point: 156-160 °C.
N-Propyl-N'-((4-fluorophenyl)sulfonyl)-3-(4-chlorophenyl)-4,5-dihydro-4-phenyl-1H-pyrazole-1-carboxamidine. Melting point: 129-138 °C.
N-(2-Propyl)-N'-((4-fluorophenyl)sulfonyl)-3-(4-chlorophenyl)-4,5-dihydro-4-phenyl-1H-pyrazole-1-carboxamidine. Melting point: 110-112 °C.
N-(2-Propyl)-N'-((4-chlorophenyl)sulfonyl)-3-(4-pyridyl)-4,5-dihydro-4-phenyl-1H-pyrazole-1-carboxamidine. Melting point: Amorphous.
N¹-Ethyl-N¹-methyl-N²-((4-chlorophenyl)sulfonyl)-3-(4-chlorophenyl)-4,5-dihydro-4-phenyl-1H-pyrazole-1-carboxamidine. Melting point: 184 °C.
N¹-Ethyl-N¹-methyl-N²-((4-fluorophenyl)sulfonyl)-3-(4-chlorophenyl)-4,5-dihydro-4-phenyl-1H-pyrazole-1-carboxamidine. Melting point: 173-176 °C.
N¹,N¹-Dimethyl-N²-((4-(trifluoromethyl)phenyl)sulfonyl)-3-(4-chlorophenyl)-4,5-dihydro-4-phenyl-1H-pyrazole-1-carboxamidine. Melting point: 195-196 °C.
N¹,N¹-Dimethyl-N²-((3-methylphenyl)sulfonyl)-3-(4-chlorophenyl)-4,5-dihydro-4-phenyl-1H-pyrazole-1-carboxamidine. Melting point: 195-198 °C.
N¹,N¹-Dimethyl-N²-((3-methoxyphenyl)sulfonyl)-3-(4-chlorophenyl)-4,5-dihydro-4-phenyl-1H-pyrazole-1-carboxamidine. Melting point: 204-206 °C.
N-Ethyl-N'-((4-chlorophenyl)sulfonyl)-3-(4-chlorophenyl)-4,5-dihydro-4-phenyl-1H-pyrazole-1-carboxamidine. Melting point: Amorphous.
N-Dimethylamino-N'-((4-chlorophenyl)sulfonyl)-3-(4-chlorophenyl)-4,5-dihydro-4-phenyl-1H-pyrazole-1-carboxamidine. Melting point: 155-159 °C.
N-Methyl-N'-((4-(trifluoromethyl)phenyl)sulfonyl)-3-(4-chlorophenyl)-4,5-dihydro-4-phenyl-1H-pyrazole-1-carboxamidine. Melting point: Amorphous.
N¹,N¹-Dimethyl-N²-((2-methylphenyl)sulfonyl)-3-(4-chlorophenyl)-4,5-dihydro-4-phenyl-1H-pyrazole-1-carboxamidine. Melting point:148-151 °C.
N-Methyl-N'-((2,4-difluorophenyl)sulfonyl)-3-(4-chlorophenyl)-4,5-dihydro-4-phenyl-1H-pyrazole-1-carboxamidine. Melting point: 85 °C.

### Example IV

### (-)-(4S)-N-methyl-N'-((4-chlorophenyl)sulfonyl)-3-(4-chlorophenyl)-4,5-dihydro-4-phenyl-1H-pyrazole-1-carboxamidine

(-)-(4S)-N-Methyl-N'-((4-chlorophenyl)sulfonyl)-3-(4-chlorophenyl)-4,5-dihydro-4-phenyl-1H-pyrazole-1-carboxamidine (7.16 gram, 0.0147 mol)) ([α²⁵_{D}] = - 150°, c = 0.01, MeOH) (melting point: 169-170 °C) was obtained via chiral chromatographic separation of racemic N-methyl-N'-((4-chlorophenyl)sulfonyl)-3-(4-chlorophenyl)-4,5-dihydro-4-phenyl-1H-pyrazole-1-carboxamidine (18 gram, 0.037 mol) using a Chiralpak AD, 20 µm chiral stationary phase. The mobile phase consisted of a mixture of hexane/ethanol (80/20 (v/v)) and 0.1 % ammonium hydroxide (25 % aqueous solution).

In an analogous manner the optically pure compounds listed below have been prepared from the corresponding racemates:
(-)-(4S)-N-Ethyl-N'-((4-chlorophenyl)sulfonyl)-3-(4-chlorophenyl)-4,5-dihydro-4-phenyl-1H-pyrazole-1-carboxamidine: ([α²⁵_{D}] = -126 °, c = 0.01, CHCl₃); Melting point: 172-175 °C. Stationary phase: Chiralcel OD. Mobile phase: A mixture of heptane/2-propanol (85/15 (v/v)).
(-)-(4S)-N-Dimethylamino-N'-((4-chlorophenyl)sulfonyl)-3-(4-chlorophenyl)-4,5-dihydro-4-phenyl-1H-pyrazole-1-carboxamidine: ([α²⁵_{D}] = -132 °, c = 0.01, CHCl₃); Melting point: 218-224 °C. Stationary phase: Chiralcel OD. Mobile phase: A mixture of heptane/2-propanol (85/15 (v/v)).
(-)-(4S)-N-Methyl-N'-((4-(trifluoromethyl)phenyl)sulfonyl)-3-(4-chlorophenyl)-4,5-dihydro-4-phenyl-1H-pyrazole-1-carboxamidine: ([α²⁵_{D}] = -131 °, c = 0.01, CHCl₃); Melting point: 157-160 °C. Stationary phase: Chiralcel OD. Mobile phase: A mixture of heptane/2-propanol (85/15 (v/v)).
(-)-(4S)-N¹,N¹-Dimethyl-N²-((2-methylphenyl)sulfonyl)-3-(4-chlorophenyl)-4,5-dihydro-4-phenyl-1H-pyrazole-1-carboxamidine: ([α²⁵_{D}] = -88 °, c = 0.01, MeOH); Melting point: Amorphous. Stationary phase: Chiralpak AD. Mobile phase: Ethanol.
(-)-(4S)-N-Methyl-N'-((2,4-difluorophenyl)sulfonyl)-3-(4-chlorophenyl)-4,5-dihydro-4-phenyl-1H-pyrazole-1-carboxamidine: ([α²⁵_{D}] = -129 °, c = 0.01, MeOH); Melting point: Amorphous. Chiralpak AD. Mobile phase: Methanol.

## Claims

1. The enantiomer having S configuration at the 4-position of their 4,5-dihydro pyrazole ring of a compound of formula (I) 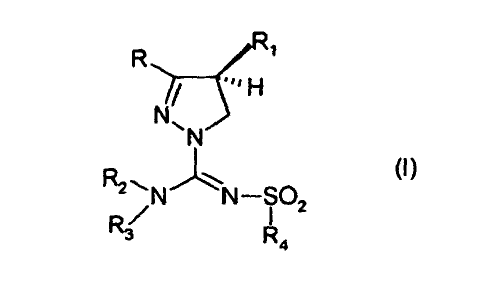 wherein
- R and R₁ are the same or different and represent 3-pyridyl or 4-pyridyl, or phenyl which may be substituted with halogen or methoxy,
- R₂ and R₃ are the same or different and represent hydrogen. alkyl (1-3 C) or dimethylamino
- R₄ represents phenyl which may be substituted with 1, 2 or 3 substituents selected from the group halogen atoms, trifluoromethyl, methoxy and alkyl (1-3 C)
and tautomers and salts thereof.

2. A compound having formula (I) as claimed in claim 1, wherein R is the group 4-chlorophenyl, R₁ is phenyl, R₂ is hydrogen, R₃ is methyl and R₄ represents 4-chlorophenyl, and salts thereof.

3. A pharmaceutical composition containing at least one compound as claimed in claim 1 as an active component.

4. A compound as daimed in claim 1, or a salt thereof, for use in medicine

5. Process for the preparation of compounds having formula I, **characterized in that** the racemic mixture of a compound having formula I is separated into the levorotatory and the dextrorotatory enantiomers.

6. Use of a compound as claimed in claim 1 for the preparation of a pharmaceutical composition for the treatment of psychiatric disorders such as psychosis, anxiety, depression, attention deficits, memory disorders and appetite disorders, obesity, neurological disorders such as Parkinson's disease, dementia, distonia, Alzheimer's disease, epilepsy, Huntington's disease, Tourette's syndrome, ischaemia, pain and other CNS diseases involving cannabinoid neurotransmission.

7. Use of a compound as claimed in claim 1 for the preparation of a pharmaceutical composition for the treatment of gastrointestinal disorders involving cannabinoid neurotransmission.

8. Use of a compound as claimed in claim 1 for the preparation of a pharmaceutical composition for the treatment of cardiovascular disorders involving cannabinoid neurotransmission.

## Patentansprüche

1. Enantiomer mit S-Konfiguration an der 4-Position des 4,5-Dihydropyrazolrings einer Verbindung der Formel (I) 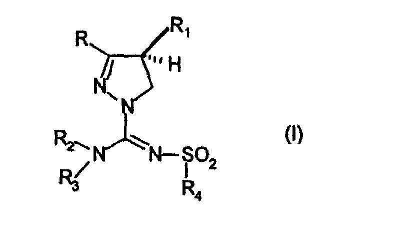 worin:
R und R₁ gleich oder unterschiedlich sind und 3-Pyridyl oder 4-Pyridyl oder Phenyl darstellen, welches mit Halogen oder Methoxy substituiert sein kann,
R₂ und R₃ gleich oder unterschiedlich sind und Wasserstoff, Alkyl (1-3 C) oder Dimethylamino darstellen,
R₄ für Phenyl steht, welches mit 1, 2 oder 3 Substituenten substituiert sein kann, ausgewählt aus der Gruppe Halogenatome, Trifluormethyl, Methoxy und Alkyl (1-3C) und Tautomere und Salze davon.

2. Verbindung mit der Formel (I) wie in Anspruch 1 beansprucht, worin R für die Gruppe 4-Chlorphenyl steht, R₁ für Phenyl steht, R₂ für Wasserstoff steht, R₃ für Methyl steht und R₄ 4-Chlorphenyl darstellt, und Salze davon.

3. Pharmazeutische Zusammensetzung, welche mindestens eine Verbindung wie in Anspruch 1 beansprucht als Wirkstoff enthält.

4. Verbindung wie in Anspruch 1 beansprucht, oder ein Salz davon, zur Verwendung in Medizin.

5. Verfahren zur Herstellung von Verbindungen mit der Formel I, **dadurch gekennzeichnet, dass** das racemische Gemisch einer Verbindung mit der Formel I in die linksdrehenden und die rechtsdrehenden Enantiomere getrennt wird.

6. Verwendung einer Verbindung wie in Anspruch 1 beansprucht zur Herstellung einer pharmazeutischen Zusammensetzung zur Behandlung von psychiatrischen Störungen wie Psychose, Angststörung, Depression, Aufmerksamkeitsdefiziten, Gedächtnisstörungen und Appetitstörungen, Fettsucht, neurologischen Störungen wie Parkinson-Erkrankung, Demenz, Dystonie, Alzheimer-Erkrankung, Epilepsie, Huntington-Erkrankung, Tourette-Syndrom, Ischämie, Schmerz und anderen CNS-Störungen, welche Cannabinoid-Neurotransmission einbeziehen.

7. Verwendung einer Verbindung wie in Anspruch 1 beansprucht für die Herstellung einer pharmazeutischen Zusammensetzung zur Behandlung von gastrointestinalen Störungen, welche Cannabinoid-Neurotransmission einbeziehen.

8. Verwendung einer Verbindung wie in Anspruch 1 beansprucht für die Herstellung einer pharmazeutischen Zusammensetzung zur Behandlung von kardiovaskulären Störungen, welche Cannabinoid-Neurotransmission einbeziehen.

## Revendications

1. Enantiomère ayant la configuration S en position 4 du noyau 4,5-dihydropyrazole d'un composé de fonnule (I) 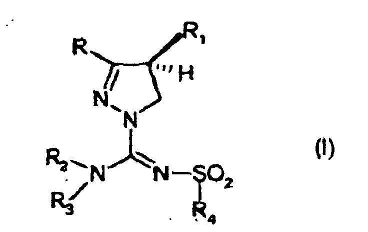 dans laquelle
- R et R₁ sont identiques ou différents et représentent un groupe 3-pyridyle ou 4-pyridyle ou un noyau phényle qui peut être substitué par un halogène ou un groupe méthoxy,
- R₂ et R₃ sont identiques ou différents et représentent un atome d'hydrogène, un groupe alkyle (en C₁-C₃) ou diméthylamino,
- R₄ représente un noyau phényle qui peut être substitué par 1, 2 ou 3 substituants choisis dans le groupe constitué par les atomes d'halogène, les groupes trifluorométhyle, méthoxy et alkyle (en C₁-C₃),
et les énantiomères et sels de celui-ci.

2. Composé de formule (I) tel que revendiqué dans la revendication 1, dans laquelle R est te groupe 4-chlorophényle, R₁ est un noyau phényle, R₂ est un atome d'hydrogène, R₃ est un groupe méthyle et R₄ représente un groupe 4-chlorophényle, et les sels de celui-ci.

3. Composition pharmaceutique contenant au moins un composé tel que revendiqué dans la revendication 1 en tant que composant actif.

4. Composé tel que revendiqué dans la revendication 1, ou un sel de celui-ci, à utiliser en médecine.

5. Procédé pour la préparation de composés de formule I, **caractérisé en ce que** le mélange racémique d'un composé de formule I est séparé pour obtenir les énantiomères lévogyre et dextrogyre.

6. Utilisation d'un composé tel que revendiqué dans la revendication 1 pour la préparation d'une composition pharmaceutique destinée au traitement de troubles psychiatriques tels que la psychose, l'anxiété, la dépression, les déficits de l'attention, les troubles de la mémoire et les troubles de l'appétit, de l'obésité, de troubles neurologiques tels que la maladie de Parkinson, la démence, la dystonie, la maladie d'Alzheimer, l'épilepsie, la maladie de Huntington, le syndrome Gilles de La Tourette, l'ischémie, la douleur et d'autres maladies du SNC impliquant la neurotransmission des cannabinoïdes.

7. Utilisation d'un composé tel que revendiqué dans la revendication 1 pour la préparation d'une composition pharmaceutique destinée au traitement de troubles gastro-intestinaux impliquant la neurotransmission des cannabinoïdes.

8. Utilisation d'un composé tel que revendiqué dans la revendication 1 pour la préparation d'une composition pharmaceutique destinée au traitement de troubles cardio-vasculaires impliquant la neurotransmission des cannabinoïdes.
